(19) Europäisches Patentamt
European Patent Office
Office européen des brevets

(11) EP 3 200 793 B1

(12) **EUROPEAN PATENT SPECIFICATION**

(45) Date of publication and mention of the grant of the patent:
**28.08.2019 Bulletin 2019/35**

(21) Application number: **15847897.4**

(22) Date of filing: **02.10.2015**

(51) Int Cl.:
*A61K 31/46* (2006.01) *A61K 9/00* (2006.01)
*A61P 39/02* (2006.01)

(86) International application number:
**PCT/US2015/053713**

(87) International publication number:
**WO 2016/054504 (07.04.2016 Gazette 2016/14)**

(54) **ATROPINE SULFATE RAPIDLY-DISINTEGRATING SUBLINGUAL TABLETS, METHODS FOR MANUFACTURE, AND METHODS FOR USE THEREOF FOR TREATMENT OF ACUTE ORGANOPHOSPHATE TOXICITY**

SCHNELL ZERFALLENDE SUBLINGUALE SULFAT-ATROPIN-TABLETTEN, VERFAHREN ZUR HERSTELLUNG UND VERFAHREN ZUR VERWENDUNG DAVON BEI DER BEHANDLUNG VON AKUTER ORGANOPHOSPHATTOXIZITÄT

COMPRIMÉS SUBLINGUAUX À DÉSINTÉGRATION RAPIDE DE SULFATE D'ATROPINE, PROCÉDÉS DE FABRICATION, ET PROCÉDÉ POUR LEUR UTILISATION POUR LE TRAITEMENT D'UNE TOXICITÉ AIGUË AUX ORGANOPHOSPHATES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.10.2014 US 201462058722 P**

(43) Date of publication of application:
**09.08.2017 Bulletin 2017/32**

(73) Proprietor: **Nova Southeastern University**
**Fort Lauderdale, CA 33314-7796 (US)**

(72) Inventors:
- **RAWAS-QALAJI, Mutasem**
  **Fort Lauderdale, FL 33314-7796 (US)**
- **AODAH, Alhussain**
  **Fort Lauderdale, FL 33314-7796 (US)**

(74) Representative: **Charrier Rapp & Liebau Patentanwälte PartG mbB**
**Fuggerstrasse 20**
**86150 Augsburg (DE)**

(56) References cited:
**WO-A1-2011/080706 US-A1- 2006 045 865**
**US-A1- 2007 059 361 US-A1- 2009 263 476**

- **RAJPAL S ET AL: "Clinical and bioavailability studies of sublingually administered atropine sulfate", AMERICAN JOURNAL OF EMERGENCY MEDICINE, CENTRUM PHILADELPHIA, PA, US, vol. 28, no. 2, 1 February 2010 (2010-02-01), pages 143-150, XP026902374, ISSN: 0735-6757, DOI: 10.1016/J.AJEM.2008.10.025 [retrieved on 2010-02-12]**
- **BRADFORD J. BOWLS ET AL: "Oral Treatment of Organophosphate Poisoning in Mice", ACADEMIC EMERGENCY MEDICINE, vol. 10, no. 3, 1 March 2003 (2003-03-01), pages 286-288, XP055424795, US ISSN: 1069-6563, DOI: 10.1111/j.1553-2712.2003.tb02006.x**
- **BOWLS ET AL.: 'Oral Treatment of Organophosphate Poisoning in Mice.' ACADEMIC EMERGENCY MEDICINE. vol. 10, no. 3, 01 March 2003, pages 286 - 288, XP055424795 DOI: 10.1111/J.1553-2712.2003.TB02006.X Retrieved from the Internet: <URL:http://onlinelibrary.wiley.com/doi/10.1197/aemj.10.3.286/pdf> [retrieved on 2015-11-05]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 200 793 B1

## Description

### FIELD OF THE INVENTION

The invention generally relates to the manufacture and evaluation of pharmaceutical compositions, particularly to the manufacture and evaluation of orally-disintegrating tablets for sublingual administration, and most particularly to an atropine sulfate (AS) rapidly-disintegrating sublingual tablet (RDST) in a sublingual dosage for treatment of organophosphate (OP) exposure and acute toxicity.

### BACKGROUND

An organophosphate (abbreviated as "OP" or "OPs" for organophosphates) or phosphate ester is the general term for esters of phosphoric acid. The general chemical structure is as follows:

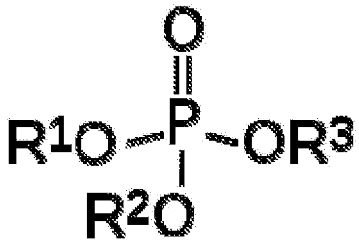

(entry for "*Organophosphate*" accessed from the Wikipedia website on September 5, 2014). Organophosphates constitute a large group of chemical compounds as there are many different substitutes of phosphoric acid esters (Newmark J. Neurology 62:1590-1596 2004).

Organophosphates are highly toxic and thus form the basis of many pesticides, insecticides, herbicides, and nerve agents (entry for "*Organophosphate*" accessed from the Wikipedia website on September 5, 2014). Organophosphate poisoning results from exposure to organophosphates, which cause inhibition of the enzyme acetylcholinesterase (also called acetylcholine esterase) (entry for "*Organophosphate Poisoning*" accessed from the Wikipedia website on September 5, 2014). Organophosphates phosphorylate the serine hydroxyl residue of acetylcholine esterase to form a covalent bond with acetylcholine esterase, thus inhibiting it irreversibly (entry for "*Organophosphate Poisoning*" accessed from the Wikipedia website on September 5, 2014 and Eddleston M. et al. Crit Care 8:R391-R7 2004). Acetylcholine esterase is critical for nerve function and its inhibition leads to accumulation of acetylcholine in the synaptic clefts between neurons. This accumulation of acetylcholine stimulates the autonomic nervous system, nicotinic receptors, and muscarinic receptors (entry for "*Organophosphate Poisoning*" accessed from the Wikipedia website on September 5, 2014 and Buckley N. et al. J Toxicol Clin Toxicol 32:61-68 1994). Overstimulation of muscarinic receptors can lead to respiratory failure and death (Eddleston M. et al. Lancet 371:597-607 2008). It is well documented that acute organophosphate toxicity is often fatal.

There are two sources of exposure to organophosphates, pesticides and nerve agents (i.e. chemical weapons). The epidemiological data clearly shows the importance of the management of organophosphate toxicity in reducing the number of causalities. According to the World Health Organization (WHO), in the "Impact of OPs on Health" report, there are approximately 3 million cases of organophosphate toxicity every year, with about 250,000 resulting in death. Worldwide, there are about 900,000 suicide cases every year; about 60% of these people poison themselves with organophosphates. In low-income countries that lack prepared medical centers, higher mortality rates have been recorded (Bertolote J. et al. The Impact of Pesticides on Health: Preventing Intentional and Unintentional Deaths from Pesticide Poisoning. In Prevention, Pesticides and Health. World Health Organization (WHO) website 2004). According to the same report, the mortality due to pesticide toxicity represents 71% of the cases in Sri Lanka, 62% in China, and 30% in India. Generally, pesticides are used excessively in all agricultural countries. In the United States alone, more than 37 types of organophosphate pesticides are used (Pesticide Usage in the United States: History, Benefits, Risks, and Trends. NC Cooperative Extension 1996; available from the North Carolina State University website). In 2011 and 2012, almost 7,000 organophosphate toxicity cases were reported according to the Annual Report of the American Association of Poison Control Centers' National Poison Data System (NPDS) (Bronstein A. et al. Clin Toxicol 50:911-1164 2012).

Following World War I, the Geneva Protocol of 1925 for the Prohibition of the Use in War of Asphyxiating, Poisonous, or Other Gases, and of Bacteriological Methods of Warfare was signed and took effect on February 8, 1928. Although this 1925 protocol prohibited the use of chemical weapons in warfare, it did not prohibit development, production, and stockpiling of chemical weapons (Weapons of Mass Destruction: Chemical Weapons. United Nations Office for Disarmament Affairs website). Following World War II, the production and stockpiling was outlawed by the Chemical

Weapons Convention of 1993 and took effect on April 29, 1997 through the establishment of the Organization for the Prohibition of Chemical Weapons (Weapons of Mass Destruction: Chemical Weapons. United Nations Office for Disarmament Affairs website and Croddy E. et al. Weapons of Mass Destruction: An Encyclopedia of Worldwide Policy, Technology, and History. Santa Barbara, CA: ABC-CLIO 2005). Despite these disarmament attempts, nerve agents were used for the first time in 1988 during the First Gulf War between Iraq and Iran, which resulted in 40,000 deaths using sarin gas (Newmark J. Neurology 62:1590-1596 2004). The second documented use of nerve agents was in 1995 in a Tokyo subway by a terrorist group, which resulted in 13 deaths and 50 injuries (Bentur Y. et al. Clin Toxicol 44:301-306 2006). In 2013, the Assad Regime used sarin gas against the people of Syria, which resulted in 1300 deaths (Report on the Alleged Use of Chemical Weapons in the Ghouta Area of Damascus on August 21, 2013. United Nations Mission to Investigate Allegations of the Use of Chemical Weapons in the Syrian Arab Republic, United Nations website).

The conventional, basic antidote in acute organophosphate toxicity cases is atropine sulfate (AS). The chemical structure is as follows:

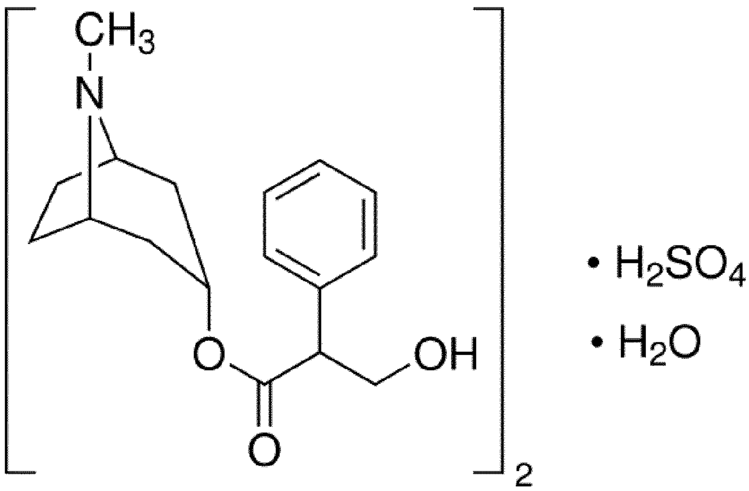

(entry from the Sigma-Aldrich company catalog, accessed online on September 5, 2014). Atropine is a competitive, muscarinic acetylcholine receptor antagonist and prevents accumulated acetylcholine from activating the receptors. Acetylcholine is the main neurotransmitter used by the parasympathetic nervous system. Generally, atropine works by countering activity of glands regulated by the parasympathetic nervous system. This countering occurs because atropine is a competitive antagonist of the muscarinic acetylcholine receptor (entry for "*Atropine*" accessed from the Wikipedia website on September 4, 2014). Atropine sulfate is capable of inhibiting the entire parasympathetic nervous system because it blocks all subtypes of muscarinic receptors. Accumulated acetylcholine is thus unable to activate the muscarinic receptors.

To date, the parenteral route is the only drug delivery route utilized for administration of atropine sulfate (AS). Atropine sulfate is administered parenterally in hospitals from a stock AS solution; or in some countries, it is given intramuscularly (IM injection) using a pre-filled auto-injector device (AtroPen®, Meridian Medical Technologies, Inc.). This pre-filled auto-injector device is designed to be used in military and community facilities (Bentur Y. et al. Clin Toxicol 44:301-306 2006). However, the AS pre-filled auto-injector device is not readily available for soldiers or civilians in most organophosphate toxicity cases. This conventional organophosphate toxicity management strategy, *i.e.* AS injection, depends on developing pre-filled auto-injectors of atropine sulfate mixed with different types of oximes (group of medications that act to re-activate acetylcholine esterase), such as pralidoxime (Eddleston M. et al. Crit Care 8:R391-R7 2004; Eddleston M. et al. Lancet 371:597-607 2008). The initial atropine sulfate (AS) dose is 2 mg, which (dose) is then doubled about every five minutes until toxicity symptoms disappear (Buckley N. et al. J Toxicol Clin Toxicol 32:61-68 1994) atropinization signs and symptoms are achieved.

Use of the auto-injector pens is associated with several drawbacks. First, the size (of the auto-injector pen) limits the number of pens that can be carried to allow for multiple dose administration (Rawas-Qalaji M. et al. J Allergy Clin Immun 117:398-403 2006). Also the high cost of the auto-injector pens limits their affordability and in turn availability to low income farmers or community facilities in low income areas at risk for organophosphate toxicity (Corcoran T. et al. J Aerosol Med Pulm D 26:46-55 2013). Furthermore, acute organophosphate toxicity is debilitating and the panic and stressful situation (when the OP toxicity occurs) limits the ability of the victim to get the auto-injector pen ready and inject himself/herself properly and effectively. In such conditions, risk for fracture of the syringe and/or occurrence of an administration error is high (Newmark J. Neurology 62:1590-1596 2004; Bentur Y. et al. Clin Toxicol 44:301-306 2006; Rawas-Qalaji M. et al. J Allergy Clin Immun 117:398-403 2006; Hague J. et al. Mil Med 169:389-391 2004).

These facts and drawbacks drove the instant inventors to look into potential alternative approaches for the treatment of organophosphate toxicity, such as new formulations for drugs administrable via the oral or sublingual drug delivery route.

The sublingual route of administration is a promising alternative for atropine sulfate (AS) administration. Tablets that disintegrate or dissolve rapidly in the patient's mouth without the use of water are convenient for the elderly, young children, patients with swallowing difficulties, and in situations where water is not available. These tablets can be quickly and effectively administered, and thus (these tablets) are particularly valuable in emergency situations such as anaphylaxis and organophosphate toxicity. For these specially designed formulations, the small volume of saliva that is available is sufficient to disintegrate or dissolve a tablet in the oral cavity. The drug released from these tablets can be absorbed partially or entirely into the systemic circulation from the buccal mucosa or sublingual cavity, or can be swallowed as a solution to be absorbed from the gastrointestinal tract.

The sublingual route usually produces a faster onset of action than traditional orally-administered tablets and the portion absorbed through the sublingual blood vessels bypasses the hepatic first pass metabolic processes (Birudaraj R. et al. J Pharm Sci 94:70-78 2005; Motwani J. et al. Clin Pharmacokinet 21(2): 83-94 1991; Ishikawa T. Chem Pharm Bull 49: 230-232 2001; Price T. et al. Obstet Gynecol 89: 340-345 1997; Kroboth P. et al. J Clin Psychopharmacol 15(4): 259-262 1995; Cunningham F. et al. J Clin Anesth 6: 430-433 1994; Scavone J. et al. Eur J Clin Pharmacol 42: 439-443 1992; Spenard J. et al. Biopharm Drug Dispos 9: 457-464 1988).

Likewise, due to high buccal and sublingual vascularity, buccally- or sublingually-delivered drugs can gain direct access to the systemic circulation and are not subject to first-pass hepatic metabolism. In addition, therapeutic agents administered via the buccal or sublingual route are not exposed to the acidic environment of the gastrointestinal tract (Mitra A. et al. Encyclopedia of Pharm Tech 2081-2095 2002). Further, the buccal and sublingual mucosae have low enzymatic activity relative to the nasal and rectal routes. Thus, the potential for drug inactivation due to biochemical degradation is less rapid and extensive than other administration routes (de Varies et al. Crit Rev Ther Drug Carr Syst. 8: 271-303 1991).

The buccal and sublingual mucosae are also highly accessible, which allows for the use of tablets which are painless, easily administered, easily removed, and easily targeted. Because the oral cavity consists of a pair of buccal mucosa, tablets, such as fast disintegrating tablets, can be applied at various sites either on the same mucosa or, alternatively, on the left or right buccal mucosa (Mitra A. et al. Encyclopedia of Pharm Tech 2081-2095 2002). In addition, the buccal and sublingual routes could be useful for drug administration to unconscious patients, patients undergoing an anaphylactic attack, or patients suffering from the effects of acute organophosphate toxicity.

Considering the high numbers of injuries and fatalities resulting from organophosphate toxicity, there is great interest and demand in the scientific community to find a better treatment for organophosphate toxicity, particularly acute organophosphate toxicity (Dolgin E. Nat Med 19:1194-1195 2013 and RamaRao G. et al. BMC Neuroscience 15:47 2014).

Atropine sulfate is an important medication in health systems worldwide for management of organophosphate toxicity. It would be very advantageous to have non-invasive sublingual drug delivery of atropine sulfate for the treatment of acute organophosphate toxicity as a potential alternative, patient-friendly, convenient, and cost-effective dosage form.

Raipal et al, American Journal of Emergency Medicine (2010) 28, 143-150, relates to clinical and bioavailability studies of sublingually administered atropine sulfate via injection.

Bredford et al., Academic Emergency Medicine 10(3), 2003, 286-288, relates to the oral treatment of organophosphate poisoning in mice.

## SUMMARY OF THE INVENTION

The extensive use of organophosphates (OPs) worldwide, as pesticides and nerve agents, has resulted in millions of injuries and fatalities due to organophosphate toxicity. The current antidote for organophosphate toxicity is a 2 mg dose of atropine sulfate (AS) delivered via intramuscular (IM) injection. This dosage is administered parenterally in hospitals from a stock AS solution or is given using a pre-filled auto-injector pen. This initial 2 mg dose of AS is repeated by doubling the initial dose amount about every five minutes until toxicity symptoms disappear or a toxic level of AS is reached. To date, parenteral administration via injection is the only route utilized for AS delivery in treatment of organophosphate toxicity.

There are many drawbacks associated with delivery of atropine sulfate (AS) via injection. First, the large size of the syringe/auto-injector pen limits the number of doses that can be conveniently carried (particularly with regard to soldiers and farmers). If the number of available doses is limited, the treatment might be insufficient for complete elimination of toxicity symptoms. Additionally, due to high cost, the pre-filled auto-injectors are generally unavailable in low income areas. Furthermore, acute organophosphate toxicity is debilitating and the panic and stressful situation (when the OP toxicity occurs) limits the ability of the victim to get the auto-injector pen ready and inject himself/herself properly and effectively. In such conditions, risk for fracture of the syringe and/or occurrence of an administration error is high. Therefore, based on these drawbacks, successful administration of atropine sulfate (AS) in organophosphate toxicity

generally requires a heath care setting.

The instant invention circumvents the aforementioned problems by providing atropine sulfate (AS) in a rapidly-disintegrating sublingual tablet (RDST) form as defined in claims 1 to 5. This AS RDST was formulated and manufactured by the instant inventors in response to the need for an alternative dosage form for atropine sulfate in treatment of organophosphate toxicity. They performed several quality control tests to ensure rapid and complete drug release and tested diffusion of atropine sulfate from the RDSTs *in vitro.*

The formulation of the AS RDST was shown to be successful. The AS RDSTs disintegrated and dissolved in < 30 seconds and 60 seconds, respectively, but without compromising hardness to pass the USP friability test. Furthermore, the diffusion of atropine sulfate from the RDSTs was similar to its diffusion from solution formulation of the same concentration. Thus, the AS RDSTs have good potential as an effective alternative dosage form for the management of organophosphate toxicity.

In one aspect, the invention provides a pharmaceutical composition including atropine sulfate (AS). In another aspect, the invention provides a therapeutic tablet including atropine sulfate (AS) as defined in the claims. In another aspect, the invention provides a pharmaceutical composition including atropine sulfate (AS) and formulated for rapid disintegration in sublingual administration as defined in the claims.

As described herein, sublingual oral disintegrating tablets (ODTs) are distinguished from conventional sublingual tablets, lozenges, or buccal tablets by the ODTs ability to rapidly fully dissolve and/or rapidly disintegrate in less than about one minute in the mouth. These tablets are described herein as "rapidly-disintegrating sublingual tablets (RDSTs)" formulated for disintegration. The term "about" in this context refers to times near or close to one minute and encompasses times in which the RDSTs can dissolve and still reasonably achieve the described function of rapidly fully dissolving and/or rapidly disintegrating in less than about one minute in the mouth.

In another aspect, the invention provides a pharmaceutical composition including atropine sulfate (AS) and at least one pharmaceutically-acceptable excipient and formulated for rapid disintegration in sublingual administration as defined in the claims.

The phrase "pharmaceutically-acceptable excipient" refers to an inactive and nontoxic substance used in association with an active substance, *i.e.* in this invention atropine sulfate, especially for aiding in the application/delivery of the active substance. Non-limiting examples of pharmaceutically-acceptable excipients are diluents, binders, disintegrants, superdisintegrants, flavorings, fillers, and lubricants. Pharmaceutically-acceptable excipients can have more than one function, a non-limiting *e.g.* a filler can also be a disintegrant. Additionally, pharmaceutically-acceptable excipients may also be referred to as non-medicinal ingredients (NMIs) or pharmaceutically-acceptable carriers.

In the composition described herein the pharmaceutically-acceptable excipients include fillers, superdisintegrants, and/or lubricants.

A filler adds volume and/or mass to a composition to facilitate precise dosage formulation and to "fill out" the size such that the composition is practical to produce and convenient for the consumer to use (entry for "*Excipient*" section 1.5 "*Fillers*" accessed from the Wikipedia website on September 23, 2014). A non-limiting example of a filler is microcrystalline cellulose.

A disintegrant is a substance or mixture of substances added to a drug formulation that facilitates the breakup or disintegration of a tablet or capsule content into smaller pieces that dissolve more rapidly than in the absence of disintegrants. A superdisintegrant increases the efficiency of disintegration by decreasing the disintegration time which in turn enhances drug dissolution rate. *See* Mangal, M. et al. International Journal of Pharmacy and Pharmaceutical Science Research 2(2):26-35 2012 for a review of disintegrants and superdisintegrants. A non-limiting example of a superdisintegrant is hydroxypropyl cellulose. The hydroxypropyl cellulose can be low substituted.

A lubricant is a substance that prevents ingredients of a drug formulation from clumping together and from sticking to the tablet punches and dies. Lubricants also ensure that tablet formation and ejection can occur with low friction between the solid and die wall (entry for "*Excipient*" section 1.8 "*Lubricants*" accessed from the Wikipedia website on September 23, 2014). A non-limiting example of a lubricant is magnesium stearate.

The invention also encompasses a pharmaceutical composition as defined above having a formulation including from about 1 to about 20 wt% of atropine sulfate, from about 20 to about 95 wt% of microcrystalline cellulose, from about 1 to about 15 wt% hydroxypropyl cellulose, and from about 0.5 to about 3 wt% magnesium stearate. The hydroxypropyl cellulose can be low substituted.

In the two immediately-preceding embodiments, the term "about" refers to near or close to the disclosed quantities and encompasses quantities in which the composition can be formulated with and still reasonably achieve the described function of rapidly fully dissolving and/or rapidly disintegrating in less than about one minute in the mouth.

A method for manufacturing atropine sulfate (AS) tablets formulated for rapid disintegration in buccal or sublingual administration is provided. The manufacturing method includes weighing atropine sulfate and pharmaceutically-acceptable excipients; sieving the atropine sulfate and pharmaceutically-acceptable excipients; combining and mixing the atropine sulfate and pharmaceutically-acceptable excipients to form a directly compressible formulation; and directly compressing the formulation to form the atropine sulfate tablets. The formulation can be directly compressed using

concave punches and dies.

The manufacturing method for atropine sulfate tablets formulated for rapid disintegration in sublingual administration encompasses an embodiment in which the filler is microcrystalline cellulose, the superdisintegrant is hydroxypropyl cellulose (can be low substituted), and the lubricant is magnesium stearate. In this embodiment the combining and mixing the atropine sulfate and pharmaceutically-acceptable excipients to form a directly compressible formulation includes the steps of: a) combining the atropine sulfate with the microcrystalline cellulose and mixing to form a mixture; b) adding two thirds of the hydroxypropyl cellulose to the mixture formed in step a) and mixing for about four minutes to form a mixture; c) mixing the magnesium stearate and the remaining one third of the hydroxypropyl cellulose to form a second mixture; and d)adding the second mixture to the mixture formed in step b) and mixing for about thirty seconds. This method achieves both internal and external positioning of the superdisintegrant.

In another aspect, the invention provides a method for treating symptoms of exposure to organophosphates (OPs) in a subject in need thereof. Prolonged exposure or exposure to excessive amounts of OPs can result in severe, acute organophosphate toxicity. The term "subject" includes any human being or animal exhibiting symptoms of exposure to organophosphates (OPs) and thus in need of treatment of the symptoms. The term "patient" is also used herein to refer to the subject. A subject could be exposed to organophosphates through pesticides or nerve agents. In addition to accidental exposure, exposure can be intended. Numerous suicides by organophosphate toxicity have been documented.

Organophosphates inhibit acetylcholine esterase, such that acetylcholine accumulates at nerve synapses and neuromuscular junctions. This accumulation of acetylcholine stimulates/activates muscarinic receptors and nicotinic receptors to stimulate the central nervous system (CNS). Thus, a patient (subject) exposed to organophosphates can exhibit any symptom resulting from excessive acetylcholine activity. The presentation of organophosphate exposure/poisoning depends upon whether the exposure/poisoning is mild, moderate, or severe. Symptoms accompanying mild exposure/poisoning include small or pinpoint pupils, painful, blurred vision, runny eyes, runny nose, excessive saliva, glassy eyes, headache, nausea, mild muscle weakness, localized muscle twitching, and/or mild agitation. Symptoms accompanying moderate exposure/poisoning include pinpoint pupils, conjunctival injection, dizziness, disorientation, coughing, wheezing, sneezing, drooling, excess phlegm, bronchorrhoea, bronchospasm, breathing difficulty, marked muscle twitching, tremors, fatigue, muscle weakness, vomiting, diarrhea, and/or urination. Symptoms accompanying severe exposure/poisoning include pinpoint pupils, confusion, agitation, convulsions, copious excessive secretions, cardiac arrhythmias, respiratory depression, respiratory arrest, and/or coma. Severe exposure/poisoning often proves fatal. *See* article "*Organophosphate Poisoning*" accessed from the Patient website on September 24, 2014.

The treatment method encompasses providing a composition including atropine sulfate and at least one pharmaceutically-acceptable excipient and administering the composition to the subject. The provided composition is formulated for rapid disintegration in sublingual administration. In this method, the pharmaceutically-acceptable excipients include fillers, superdisintegrants, and/or lubricants. A specific, albeit non-limiting, example includes microcrystalline cellulose as a filler, hydroxypropyl cellulose as a superdisintegrant, and magnesium stearate as a lubricant. The hydroxypropyl cellulose can be low substituted.

The method can further encompass administering additional compositions to treat symptoms of organophosphate exposure/toxicity/poisoning concomitantly with the atropine sulfate rapidly-disintegrating sublingual tablets (AS RDSTs). A non-limiting example of such a composition is a cholinesterase re-activator. A specific, albeit non-limiting, example of a cholinesterase re-activator is pralidoxime chloride.

The phrase "effective amount" refers to the amount of a composition necessary to achieve the composition's intended function.

The phase "therapeutically-effective amount" refers to the amount of a composition required to achieve the desired function, *i.e.* treatment of the symptoms of exposure to organophosphates.

The invention also encompasses atropine sulfate (AS) rapidly-disintegrating sublingual tablets (RDSTs) produced by any of the fabrication methods and/or manufacture steps described herein.

Other objectives and advantages of this invention will become apparent from the following description taken in conjunction with the accompanying drawings, wherein are set forth, by way of illustration and example, certain embodiments of this invention. The drawings constitute a part of this specification and include exemplary embodiments of the present invention and illustrate various objects and features thereof.

BRIEF DESCRIPTION OF THE DRAWINGS

A more complete understanding of the present invention may be obtained by references to the accompanying drawings when considered in conjunction with the subsequent detailed description. The embodiments illustrated in the drawings are intended only to exemplify the invention and should not be construed as limiting the invention to the illustrated embodiments.

FIG. 1 is a schematic illustration of the disintegration apparatus used for carrying out the disintegration test on the rapidly-disintegrating sublingual tablets (RDSTs).

FIGS. 2A-C are images from the wetting test on the rapidly-disintegrating sublingual tablets: FIG. 2A is an image of a dry tablet prior to the start of the test; FIG. 2B is an image of the tablet placed on a wet paper towel during the test; and FIG. 2C is an image of the wet tablet after completion of the test.

FIGS. 3A-B are images from the water uptake test on the rapidly-disintegrating sublingual tablets: FIG. 3A shows an image of a dry tablet placed in a test dish prior to the start of the test and FIG. 3B shows an image of a water-saturated tablet after completion of the test.

FIG. 4 is a graph plotting results from the *in vitro* diffusion studies on the rapidly-disintegrating sublingual tablets. The graph shows the cumulative atropine sulfate (AS) diffused per area (μg/cm2) versus time from the rapidly-disintegrating sublingual tablets (RDSTs) (n=4). The reference is 2 ml of 1mg/ml atropine sulfate (AS) solution.

FIG. 5 is also a graph plotting results from the *in vitro* diffusion studies on the rapidly-disintegrating sublingual tablets. The graph shows the atropine sulfate (AS) diffused percent (%) versus time from the rapidly-disintegrating sublingual tablets (RDSTs) (n=4). The reference is 2 ml of 1mg/ml atropine sulfate (AS) solution.

FIG. 6 is a graph plotting results from the *ex vivo* diffusion studies on the rapidly-disintegrating sublingual tablets. The graph shows the cumulative atropine sulfate (AS) diffused per area (μg/$cm^2$) versus time from the rapidly-disintegrating sublingual tablets (RDSTs) (n=4). The reference is 2 ml of 1mg/ml atropine sulfate (AS) solution.

FIG. 7 is also a graph plotting results from the *ex vivo* diffusion studies on the rapidly-disintegrating sublingual tablets. The graph shows the atropine sulfate (AS) diffused percent (%) versus time from the rapidly-disintegrating sublingual tablets (RDSTs) (n=4). The reference is 2 ml of 1 mg/ml atropine sulfate (AS) solution.

## DETAILED DESCRIPTION OF THE INVENTION

For the purpose of promoting an understanding of the principles of the invention, reference will now be made to embodiments illustrated herein and specific language will be used to describe the same. It will nevertheless be understood that no limitation of the scope of the invention is thereby intended. Any alterations and further modification in the described compositions, formulations, and methods and any further application of the principles of the invention as described herein, are contemplated as would normally occur to one skilled in the art to which the invention relates.

The worldwide use of organophosphates (OPs), as pesticides and nerve agents, has been reported as causing 3 million toxicity cases per year. According to the American Association of Poison Control Centers, almost 7000 toxicity cases were reported in the last two years in the United States alone due to organophosphate use as pesticides. The recommended antidote for organophosphate toxicity is a 2 mg injection of atropine sulfate (AS). Due to the side effects and administration limitations of injections mainly outside of healthcare settings, an alternative easy-to-use dosage form of atropine sulfate would be most beneficial.

In response to the need for an alternative dosage form for atropine sulfate, the instant inventors formulated and manufactured an exemplary formula for rapidly-disintegrating or dissolving sublingual tablets (RDSTs) of atropine sulfate (AS). They performed several quality control tests to ensure rapid and complete drug release and tested diffusion of atropine sulfate from the AS RDSTs *in vitro.* These experiments and results are described herein below.

## FORMULATION

### Powder Composition

Six tablet batches of rapidly-disintegrating sublingual tablets (RDSTs) were manufactured by direct compression and evaluated for quality control. The first and second batches were used to manufacture tablets weighing 150 mg and contain 0 mg atropine sulfate (AS) (B0), as placebo, and 2 mg AS (B1). The third to sixth batches were used to manufacture tablets weighing 50 mg and contain 0 mg (B2), as placebo, 2 mg (B3), 4 mg (B4), and 8 mg (B5) atropine sulfate (AS). These formulations are shown below in Table 1.

**Table 1**: Atropine Sulfate (AS) Rapidly-Disintegrating Sublingual Tablets (RDSTs).

| Formulations: | | | | | | | | |
|---|---|---|---|---|---|---|---|---|
| No. | Ingredient | Function | B0 | B1 | B2 | B3 | B4 | B5 |
| 1 | Atropine Sulfate | API | 0.00 (0%) | 2.00 (1.33%) | 0.00 (0%) | 2.00 (4%) | 4.00 (8%) | 8.00 (16%) |

(continued)

| Formulations: | | | | | | | | |
|---|---|---|---|---|---|---|---|---|
| No. | Ingredient | Function | B0 | B1 | B2 | B3 | B4 | B5 |
| 2 | Microcrystalline Cellulose (Ceolus® PH-301) | Filler | 133.65 (89.1%) | 130.50 (87%) | 44.55 (89.1%) | 42.75 (85.5%) | 40.95 (81.9%) | 37.35 (74.7%) |
| 3 | Low-substituted Hydroxypropyl Cellulose (L-HPC, LH-11®) | Super-disintegrating Agent | 14.85 (9.9%) | 14.50 (9.7%) | 4.95 (9.9%) | 4.75 (9.5%) | 4.55 (9.1%) | 4.15 (8.3%) |
| 4 | Magnesium Stearate | Lubricant | 1.5 (0.5%) | 3.00 (2%) | 0.50 (1.0%) | 0.50 (1%) | 0.50 (1%) | 0.50 (1%) |
| | Total Tablet Weight | | 150 (100%) | 150.0 (100%) | 50.0 (100%) | 50.0 (100%) | 50.0 (100%) | 50.0 (100%) |

Powder Mixing and Sieving

All powders were sieved before mixing using sieve number 230 (63 μm). First, atropine sulfate was mixed with microcrystalline cellulose (Asahi Kasei Chemicals Corporation, Tokyo, Japan) by a manual geometric method.

The mixing procedure was then optimized to achieve both internal and external positioning of the superdisintegrant; *i.e.* low-substituted hydroxypropyl cellulose. In the method of internal and external positioning, the superdisintegrant is divided into two portions. One portion is added before granule formation (internal) and the remaining portion is added to the granules (external) with mixing prior to compression. When both internal and external methods are used, the extragranular superdisintegrant portion breaks the tablet into granules and the granules further disintegrate to release the active substance by the intragranular disintegrant portion (Kumar G. et al. *Journal of Global Pharma Technology* 4(02):1-12 2012 and Rawas-Qalaji et al. *AAPS PharmSciTech* 7:E41 2006).

Two-thirds of the low-substituted hydroxypropyl cellulose (Shin-Etsu Chemical Company, Tokyo, Japan) was mixed with the powdered mixture of atropine sulfate and microcrystalline cellulose using a three-dimensional mixture (Inversina, Bioengineering AG, Wald, Switzerland) for four minutes. Magnesium stearate and the remaining one-third of the low-substituted hydroxypropyl cellulose was manually mixed and then added to the mixture of atropine sulfate, microcrystalline cellulose, and low-substituted hydroxypropyl cellulose to be mixed for an additional thirty seconds.

Direct Compression

The mixture was then compressed into tablets via direct compression. In direct compression an active agent, *i.e.* drug, is blended with a variety of excipients, lubricated, and compressed (Kumar G. et al. Journal of Global Pharma Technology 4(02):1-12 2012). For tablets weighing 150 mg, 5"/16" concave punches and dies were used and for tablets weighing 50 mg, 3"/16" concave punches and dies were used (Natoli Engineering Company, Inc. Saint Charles, MO).

QUALITY CONTROL TESTS

The manufactured rapidly-disintegrating sublingual tablets were tested for content uniformity (CU), weight variation (WV), and friability using the United States Pharmacopeia (USP) standard tests and limits. Due to a lack of an accurate USP test that can discriminate small differences between rapidly-disintegrating sublingual tablets (RDSTs), tablet disintegration and dissolution were tested using apparatuses (and procedures) developed by the instant inventors that are capable of detecting small differences between tablets. The wetting time (WT) and water uptake (WU) of the tablets were also tested using procedures modified by the instant inventors. *See* Shukla, D. et al. *Sci Pharma* 77:327-341 2009 for an overview of the evaluation techniques for mouth dissolving tablets.

Content Uniformity and Weight Variation Tests

According to USP, the content uniformity test is performed for tablets that contain less than 25 mg or less than 25% of the active material and be within 90-110% of the labeled amount (USB/NF. Official Monograph: Atropine Sulfate Tablet. In: United States Pharmacopeia. 37/32 ed. Rockville, MD: United States Pharmacopeia Convention, Inc. 2014,

pages 1878-1879). Both content uniformity and weight variation tests were performed according to the USP standards for all AS RDST formulations. Tablet content was analyzed by high-performance liquid chromatography (HPLC) using the standard USP procedures for analysis of atropine sulfate (AS) injection (USB/NF. Physical Tests: <905> Uniformity of Dosage Units. In: United States Pharmacopeia 37/32 ed. Rockville, MD: United States Pharmacopeia Convention, Inc. 2014, pages 491-494).

Breaking Force and Friability Tests

The AS RDSTs were tested (breaking force) according to the USP guidelines (USP/NF. General Chapters: <1217> Tablet Breaking Force. In: United States Pharmacopeia. 37/32 ed. Rockville, MD: United States Pharmacopeia Convention, Inc. 2014, pages 1146-1148) using Hardness Tester LIH-3 (Vanguard, Spring, TX).

Further, according to the standard USP Friability Test (USP/NF. General Chapters: <1216> Tablet Friability. In: United States Pharmacopeia. 37/32 ed. Rockville, MD: United States Pharmacopeia Convention, Inc. 2014, pages 1145-1146), sixty five tablets that weighed 6.5 g were tested using the USP Friability Tester (Pharma Test Apparatebau, GmbH, Hainburg, Germany).

Disintegration Test

The Disintegration Test is one of the standard USP tests. However, the current official USP standard test is unable to discriminate between small formulation differences. Thus, the instant inventors developed their own disintegration test for the AS RDSTs.

Apparatus

The disintegration apparatus used for carrying out the disintegration test on the rapidly-disintegrating sublingual tablets (RDSTs) is illustrated schematically in FIG. 1 and includes a motor 10 with a rotating shaft 12, a stainless steel round basket 14, a stainless steel wire screen at the base (bottom) and height (walls) of the basket, a glass beaker 16 containing warmed fluid 18, and a water bath 20 equipped with a thermostat 22. Non-limiting exemplary dimensions for the test set up follow. The rotating shaft has a speed rate of 40±10 rpm, a diameter of 8±2 mm, and a length of 220±20 mm. The stainless steel round basket has a diameter of 38.5±1 mm, a height of 23±2 mm, and a welded seam. The stainless wire screen at the bottom (base) of the basket is connected to the rotating shaft at its center and has apertures of 0.36-0.44 mm and a wire diameter of 0.22-0.31 mm. The glass beaker has a diameter of 70±10 mm, a height of 90±10 mm, and a volume of 350 ml. In use, the glass beaker contains 150±5 ml of a suitable warmed fluid; *i.e.* water at a temperature of 37±2 C°. The fluid level in the water bath has a height of about 100 mm. In use, the basket is partially submerged in the warmed fluid to a depth of about 10±2 mm and then the basket is rotated to facilitate table disintegration.

Example Test Procedure

1. Immerse the stainless steel basket to a depth of 10 mm into the warmed fluid.
2. Rotate the basket at a speed of 40 rpm.
3. Randomly select six tablets to test.
4. Drop one tablet (one dosage unit) at a time into the rotating basket.
5. Record the time required (in seconds) for complete disintegration of the tablet.

Six tablets are selected to test based on the similar number of tablets used in the official USP standard disintegration test. The apparatus can additionally include a built-in stop that starts and stops automatically through connection to an attached sensor. The times can also be recorded using a manual stop watch.

Disintegration does not necessarily imply complete solution of the tablet. According to the USP standards, complete disintegration is defined as that state in which any residue of the tablet, except fragments of insoluble ingredients and/or coating, remaining on the wire screen is a soft mass having no palpably firm core (U.S. Pharmacopeia, General Chapter 701 "Disintegration" accessed from the internet on September 17, 2014).

Wetting Time (WT)

The tablet wetting time was measured according to a previously modified non-USP Wetting Test (WT) designed to simulate the low fluid volume and static motion available in the oral cavity and to better discriminate between small

differences in formulation in the presence of limited fluid volume (Rawas-Qalaji M. et al. Drug Dev Ind Pharm 33:523-530 2007; Rawas-Qalaji M. et al. AAPS PharmSciTech 7:E41 2006).

Six tablets were randomly selected and tested. One tablet was placed on double folded tissue paper in a dish having about 6ml of water. The time for complete wetting of the tablet is recorded as the wetting time (Shukla, D. et al. Sci Pharm 77:327-341 2009). The Wetting Test is illustrated in FIGS. 2A-C: FIG. 2A is an image of a dry tablet prior to the start of the test; FIG. 2B is an image of the tablet placed on a wet paper towel during the test; and FIG. 2C is an image of the wet tablet after completion of the test.

Dissolution Test

Dissolution of the tablets was measured according to a previously modified non-USP dissolution test designed to simulate the low fluid volume and static motion available in the oral cavity and to better discriminate between small differences in formulation in the presence of limited fluid volume (Rachid O. et al. AAPS PharmSciTech 12:544-552 2011).

Dissolution testing is a means of identifying and proving the availability of active drug materials in their delivered form. A dissolution test simulates the availability of active substance and allows prediction of the time for complete release of the material from the dosage form (Tablet Dissolution in the section "Analytical Products" accessed from the website of Pharma Test on September 17, 2014).

Six tablets were randomly selected and tested. Drug released and dissolved from each tablet after 60 seconds was analyzed by High Performance Liquid Chromatography (HPLC) using the standard USP procedure for analyzing atropine sulfate (AS) injections (USP/NF. Official Monograph: Atropine Sulfate Injection. In: United States Pharmacopeia. 37/32 ed. Rockville, MD: United States Phamacopeial Convention, Inc. 2014, pages 1875-1876).

Water Uptake (WU) Test

Water uptake was measured in order to test the ability of the tablet to uptake and hold water to swell and dissolve the atropine sulfate (AS) within the tablet. The volume of water up-taken by each tablet was measured by weighing the tablet before and after addition of the water droplets. To test the tablets, water was added dropwise, using a glass pipette, to the top of a pre-weighted tablet placed on an analytical balance (d=0.01 mg) until water starts oozing outside the tablet because the tablet cannot hold any more water. The Water Uptake Test is illustrated in FIGS. 3A-B: FIG. 3A shows an image of a dry tablet placed in a test dish prior to the start of the test and FIG. 3B shows an image of a water-saturated tablet after completion of the test. The percentage of water uptake was determined using the following equation:

$$\text{Water Uptake \%} = (\text{weight of wet tablet} - \text{weight of dry tablet}) \times 100/\text{weight of dry tablet}.$$

The resulting data obtained from the quality control tests of manufactured AS RDSTs are tabulated below:

**Table 2**: Quality Control Tests of Atropine Sulfate (AS) Rapidly-Disintegrating Sublingual Tablets (RDSTs): Placebo Tablet Weighing 150 mg (B0); 2 mg AS Tablet Weighing 150 mg (B1); Placebo Tablet Weighing 50 mg (B2); 2 mg AS Tablet Weighing 50 mg (B3); 4 mg AS Tablet Weighing 50 mg (B4); and 8 mg AS Tablet Weighing 50 mg (B5):

| Tablet | B0 | B1 | B2 | B3 | B4 | B5 |
|---|---|---|---|---|---|---|
| Diameter | 7.95 ± 0.0 | 7.95 ± 0.0 | 4.77 ± 0.006 | 4.77 ± 0.004 | 4.76 ± 0.005 | 4.76 ± 0.004 |
| Content Uniformity (%) | N/A | 101 ± 5.6 | N/A | 98.06 ± 2.2 | 93.17 ± 2.1 | 99.96 ± 1.5 |
| Breaking Force (KF) | 2.94 ± 0.14 | 2.7 ± 0.19 | 3.18 ± 0.56 | 3.28 ± 0.45 | 2.78 ± 0.12 | 1.45 ± 0.05 |
| Friability (loss %) | 0% | 0.70% | 0% | 0.32% | 0.14% | 0.09% |
| Disintegration Time (sec) | 25.19 ± 0.39 | 20.67 ± 1.15 | 15.92 ± 0.92 | 14.52 ± 1.58 | 12.14 ± 2.12 | 13.93 ± 1.29 |
| Drug Dissolved (%)§ | N/A | 87.8% ± 10 | N/A | 98.07 ± 4.08 | 100.0% ± 4.7 | 88.48 ± 14 |
| Wetting Time (sec) | 14.76 ± 4.05 | 7.56 ± 0.7 | 7.4 ± 0.2 | 7.2 ± 0.35 | 16.7 ± 0.8 | 11 ± 0.9 |

(continued)

| Tablet | B0 | B1 | B2 | B3 | B4 | B5 |
|---|---|---|---|---|---|---|
| Water Uptake (%) | 330.3 ± 10.7 | 296.19 ± 2.8 | 272.2 ± 13.3 | 292.6 ± 7.5 | 275.4 ± 4.3 | 230 ± 12 |
| * Results are presented as mean ± SD<br>§ Drug dissolution was measured for 60 seconds. | | | | | | |

In a further example, six rapidly-disintegrating sublingual tablets (RDSTs) batches containing 0mg (placebo), 2mg, and 4mg atropine sulfate (AS) were manufactured by direct compression. Tablet weights were 150mg or 50mg for placebo and 2 mg AS tablets. The formulation included microcrystalline cellulose, low-substituted hydroxypropyl cellulose, magnesium stearate, and AS. Pre-sieved powders (mesh no. 230) were mixed by three-dimensional mixer and compressed using 5/16" or 3/16" bevel punches and dies. Quality control tests including USP friability, weight variation (WV), and content uniformity (CU) tests; and non-USP disintegration, wetting time (WT), water uptake (WU), and dissolution tests were used for all formulated batches (Table 2).

DIFFUSION STUDIES

*In Vitro* and *Ex Vivo* Diffusion of Atropine Sulfate

Evaluation of the *in vitro* and *ex vivo* diffusion of atropine sulfate (AS) from the AS RDSTs was performed using static vertical jacketed Franz cells with an OD of 20 mm and a reservoir volume of 20±1 ml (PermeGear Inc. Hellertown, PA). For the *in vitro* studies, a cellulose dialysis membrane, 7 Spectra/Por® with a 1000 Dalton MWCO (Spectrum Laboratories Inc. Rancho Dominguez, CA) was used as the diffusional membrane. For the *ex vivo* studies, an excised porcine sublingual membrane (PSM) was used as the diffusional membrane. PSM was documented to be structurally and physiologically similar to human sublingual membrane (Rawas-Qalaji MM, Simons FER, Simons KJ. Fast-disintegrating sublingual tablets: Effect of epinephrine load on tablet characteristics. AAPS PharmSciTech. 2006: 7(2):E72-E8; Collins P, Laffoon J, Squier C, editors. Comparative structure of porcine oral epithelium. Journal of Dental Research; 1981; Birudaraj R, Berner B, Shen S, Li X. Buccal permeation of buspirone: mechanistic studies on transport pathways. Journal Of Pharmaceutical Sciences. 2005;94(1):70-8.; Ong CMY, Heard CM. Permeation of quinine across sublingual mucosa, in vitro. International Journal of Pharmaceutics. 2009;366(1-2):58-64). Frozen lower jaws of male adult domestic pigs (*Sus domesticus*) were obtained from a local butcher shop and thawed at room temperature then dissected to separate the epithelial sublingual membrane at the floor of the mouth from underneath connective tissues. The dissected membranes were inspected visually for integrity and frozen again at -20° C. Membranes were thawed at room temperature to be used before each experiment. A receptor chamber with a magnetic stirrer, filled with phosphate buffer (ranging in pH from 5.0 to about 6.5), was used as the diffusion medium. This test simulates sublingual conditions as pH 5.8 is the average pH of saliva.

Before beginning each experiment air bubbles were removed after mounting the membrane between the donor and receptor chambers. The water bath was set at 37 °C and water was circulated in the jacketed Franz cells. The mounted membranes were equilibrated with the diffusion medium from both sides for 30 minutes and were checked for leaks.

Each tablet tested was at the center of the donor chamber on the membrane at T0 and then 2 ml of the diffusion medium was added to facilitate tablet disintegration and dissolution. Aliquots of 200 μl were withdrawn from the receptor chamber using 6-inch needles (Popper & Sons, Inc. New Hyde Park, NY) and 1 ml syringes at 5, 10, 15, 20, 30, 45, 60, 75, and 90 minute time intervals. The volumes withdrawn were replenished with fresh medium. Samples were transferred to High Performance Liquid Chromatography (HPLC) vials for HPLC analysis using an ultra violet (UV) detector according to the standard USP method for analyzing atropine sulfate injections (USP/NF. Official Monograph: Atropine Sulfate Injection. In: United States Pharmacopeia. 37/32 ed. Rockville, MD: United States Phamacopeial Convention, Inc. 2014, pages 1875-1876). A 2 ml solution, 1 mg/ml, of atropine sulfate was used as a reference.

Statistical Analysis

The mean±SD cumulative diffused atropine sulfate (AS) per area ($\mu g/cm^2$) and percentage of diffused AS for each rapidly-disintegrating sublingual (RDST) tablet formulation were calculated. The mean±SD AS influx, *J* ($\mu g/cm^2$/minute), and lag time, tL (minutes) were calculated from the slope and the intercept with the x-axis of each graph (n=4). Also, AS permeability, *P* (cm/minute), was calculated by dividing *J* by AS concentration in the donor chamber at $T_0$. The area under the curve of diffused AS per area (cumulative diffused AS per area versus time), $JAUC_{0-90}$

($\mu$g/$cm^2$/minute); the maximum AS diffused, *J*max ($\mu$g/$cm^2$); and the time to reach *J*max, Tmax (minutes) were calculated using WinNonlin software (Pharsight Mountain View, CA). Data were statistically compared by one-way ANOVA and Tukey-Kramer tests using NCSS statistical software (NCSS Kaysville, UT). Differences were considered to be statistically significant at $p< 0.05$.

*In Vitro* and *Ex Vivo* Diffusion Study Results

The cumulative atropine sulfate (AS) diffused *in vitro* per area ($\mu$g/$cm^2$) versus time and percentage of *in vitro* diffused AS for each rapidly-disintegrating sublingual tablet (RDST) formulation are shown in FIGS. 4 and 5, respectively.

FIG. 4 is a graph plotting results from the *in vitro* diffusion studies on the tablets. The graph shows the cumulative atropine sulfate (AS) diffused per area ($\mu$g/cm2) versus time from the rapidly-disintegrating sublingual tablets (RDSTs) (n=4). The reference is 2 ml of 1mg/ml atropine sulfate (AS) solution.

FIG. 5 is also a graph plotting results from the *in vitro* diffusion studies on the tablets. The graph shows the atropine sulfate (AS) diffused percent (%) versus time from the rapidly-disintegrating sublingual tablets (RDSTs) (n=4). The reference is 2 ml of 1mg/ml atropine sulfate (AS) solution.

The cumulative atropine sulfate (AS) diffused *ex vivo* per area ($\mu$g/$cm^2$) versus time and percentage of *ex vivo* diffused AS for each rapidly-disintegrating sublingual tablet (RDST) formulation are shown in FIGS. 6 and 7, respectively.

FIG. 6 is a graph plotting results from the *ex vivo* diffusion studies on the rapidly-disintegrating sublingual tablets. The graph shows the cumulative atropine sulfate (AS) diffused per area ($\mu$g/$cm^2$) versus time from the rapidly-disintegrating sublingual tablets (RDSTs) (n=4). The reference is 2 ml of 1mg/ml atropine sulfate (AS) solution.

FIG. 7 is also a graph plotting results from the *ex vivo* diffusion studies on the rapidly-disintegrating sublingual tablets. The graph shows the cumulative atropine sulfate (AS) diffused percent (%) versus time from the rapidly-disintegrating sublingual tablets (RDSTs) (n=4). The reference is 2 ml of 1 mg/ml atropine sulfate (AS) solution.

Four formulations and the reference sample of atropine sulfate were tested (Table 3). The four formulations were as follows: B1, 2 mg AS with 150 mg tablet weight; B3, 2 mg AS with a 50 mg tablet weight; B4, 4 mg AS with a 50 mg tablet weight; and B5, 8 mg AS with a 50 mg tablet weight.

For *in vitro* diffusion, mean $\pm$ SD $JAUC_{0-90}$ of diffused AS and influx (*J*) of B5 (47485.9 $\pm$ 7846.7 $\mu$g/$cm^2$/min, 20.0 $\pm$ 6.48 $\mu$g/$cm^2$/min, respectively) were significantly higher ($p < 0.05$) than B4 (30103.8 $\pm$ 4248.9 $\mu$g/$cm^2$/min, 10.3 $\pm$ 3.5 $\mu$g/$cm^2$/min, respectively), which were also significantly higher ($p < 0.05$) than B3 (9488.0 $\pm$ 1907.5 $\mu$g/$cm^2$/min, 4.2 $\pm$ 1.3 $\mu$g/$cm^2$/min, respectively), B1 (8063.8 $\pm$ 1211.39 $\mu$g/$cm^2$/min, 2.5 $\pm$ 0.5 $\mu$g/$cm^2$/min, respectively), and the reference (11387.8 $\pm$ 468.5 $\mu$g/$cm^2$/min, 3.3 $\pm$ 0.3 $\mu$g/$cm^2$/min, respectively). However, $JAUC_{0-90}$ and J for B3 were not significantly different ($p > 0.5$) from the reference. Results are shown below in Table 3.

**Table 3**: *In Vitro* Diffusion of Atropine Sulfate (AS) Rapidly-Disintegrating Tablets (RDSTs); 2 mg AS Tablet Weighing 150 mg (B1); 2 mg AS Tablet Weighing 50 mg (B3); 4 mg AS Tablet Weighing 50 mg (B4); 8 mg AS Tablet Weighing 50 mg (B5); and reference, 2 ml of 1mg/ml solution

| | **B1** | **B3** | **B4** | **B5** | **Reference** |
|---|---|---|---|---|---|
| $JAUC_{0-90}$ ($\mu$g/$cm^2$/min) | 7867.5 $\pm$ 1251.3 | 9488.0 $\pm$ 1907.5 | 30103.8 $\pm$ 4248.9¥ | 47485.9 $\pm$ 7846.7§ | 11387.8 $\pm$ 468.5 |
| *J* ($\mu$g/$cm^2$/min) | 2.5 $\pm$ 0.5 | 4.1 $\pm$ 1.3£ | 10.3 $\pm$ 3.5¥ | 20 $\pm$ 6.48§ | 3.3 $\pm$ 0.3 |
| P (cm/min) | 1.3 $\pm$ 0.3 | 2.1 $\pm$ 0.7 | 2.6 $\pm$ 0.9 | 2.5 $\pm$ 0.81 | 1.63 $\pm$ 0.15 |
| $t_L$ (min) | 1.8 $\pm$ 2.1 | 0.5 $\pm$ 1.0 | 0.25 $\pm$ 0.5 | 2.0 $\pm$ 2.82 | 1.81 $\pm$ 0.85 |

* Results are presented as mean $\pm$ SD, $JAUC_{0-90}$, *J*:influx, *P*:permeability, $t_L$ :lag time
§ $p< 0.05$
¥ $p < 0.05$, from B3 and B1
£ $p < 0.05$, from B1

For *ex vivo* diffusion, mean $\pm$ SD $JAUC_{0-90}$ of diffused AS and influx (J) of B5 (8849 $\pm$ 680 $\mu$g/$cm^2$/min, 3.89 $\pm$ 1.38 $\mu$g/$cm^2$/min) were significantly higher (p= <0.05) than B4 (4557 $\pm$ 761.3 $\mu$g/$cm^2$/min, 1.57 $\pm$ 0.69 $\mu$g/$cm^2$/min), B3 (2296 $\pm$ 761.3 $\mu$g/$cm^2$/min, 1.22 $\pm$ 0.54 $\mu$g/$cm^2$/min), B1 (2473.5 $\pm$ 894.5 $\mu$g/$cm^2$/min, 0.953 $\pm$ 0.36 $\mu$g/$cm^2$/min), and the reference solution (2292 $\pm$ 761.3 $\mu$g/$cm^2$/min, 0.92 $\pm$ 0.28 $\mu$g/$cm^2$/min). Results are shown below in Table 4.

**Table 4**: *Ex Vivo* Permeation of Atropine Sulfate (AS) Rapidly-Disintegrating Sublingual Tablets (RDSTs): 2 mg AS Tablet Weighing 150 mg (B1); 2 mg AS Tablet Weighing 50 mg (B3); 4 mg AS Tablet Weighing 50 mg (B4); 8 mg AS Tablet Weighing 50 mg (B5); and reference, 2 ml of 1 mg/ml solution.

| | **B1** | **B3** | **B4** | **B5** | **Reference** |
|---|---|---|---|---|---|
| $JAUC_{0-90}$ ($\mu g/cm^2/min$) | 2473.53 ± 894.54 | 2296 ± 761.3 | 4557 ± 761.3¥ | 8849 ± 680.9§ | 2292 ± 761.3 |
| Diffusion (%) | 6.29 ± 2.45 | 10.26 ± 4.57 | 4.58 ± 1.22 | 8.95 ± 3.8 | 10.39 ± 4.1 |
| J ($\mu g/cm^2/min$) | 0.9525 ± 0.36 | 1.22 ± 0.54 | 1.57 ± 0.69 | 3.894 ± 1.38§ | 0.9191 ± 0.28 |
| P (cm/min) | 0.47 ± 0.18 | 0.61 ± 0.27 | 0.39 ± 0.17 | 0.49 ± 0.17 | 0.4 ± 0.1 |
| $t_L$ (min) | 0 ± 0 | 5.75 ± 3.3 | 7.25 ± 6.75 | 7.8 ± 6.25 | 0 ± 0 |
| * Results are presented as mean ± SD, $JAUC_{0-90}$, *J*:influx, *P*:permeability, $t_L$ :lag time<br>§ p< 0.05<br>¥ p < 0.05, from B3 and B1 | | | | | |

Effect of Tablet Dimension and Weight on the Influx of Atropine Sulfate

The effect of tablet dimension and weight on the influx of atropine sulfate (AS) from the rapidly-disintegrating sublingual tablets (RDSTs) was also investigated.

Two batches of AS 2mg RDSTs were manufactured by direct compression (Table 1). The first AS RDSTs batch was compressed using 3/16" bevel punches and dies to make 50 mg tablet weight. The second AS RDSTs batch was compressed using 5/16" bevel punches and dies to make 150 mg tablet weight. The *in vitro* diffusion of AS 2 mg RDSTs weighted 50 mg (n=4) and 150 mg (n=8) were performed using Franz cells through cellulose membrane (MWCO 1000D). Phosphate buffer, pH 6.5, was used as the diffusion media. AS RDST was placed on the cellulose membrane in the donor chamber, which contained 2 ml of phosphate buffer. Aliquot samples, 200μl, were withdrawn from the receptor chamber at preset time intervals.

Mean ± SD thickness and diameter of AS 2mg RDSTs weighted 50 mg and 150 mg were 3.1 ± 0.0 mm and 4.77 ± 0.00 mm, and 4.0 ± 0.0 mm and 7.95 ± 0.0 mm, respectively. Mean ± SD AS influx (*J*) from RDSTs weighted 50 mg (4.1 ± 1.3 μg/cm2/minute) was significantly higher ($p$ <0.05) than RDSTs weighted 150 mg (2.5 ± 0.5 μg/cm2/minute). However, AS permeability was not significantly different (p >0.05) between RDSTs weighted 50 mg and 150 mg, 2.1 ± 0.7 cm/minute and 1.3 ± 0.3 cm/minute, respectively. Thus, the tablet dimensions and weight of atropine sulfate (AS) 2 mg RDSTs can affect the influx of AS.

Conclusion

In conclusion, the formulation of atropine sulfate (AS) as rapidly-disintegrating sublingual tablets (RDSTs) was shown to be successful. AS RDSTs disintegrated and dissolved in < 30 seconds and 60 seconds without compromising hardness to pass the USP friability test.

The diffusion of atropine sulfate from the RDSTs was similar to its diffusion from solution formulation of the same concentration, which indicates that diffusion of atropine sulfate was not delayed by the RDST formulation. Increasing the atropine sulfate dose significantly increases its diffusion. Furthermore, the tablet dimension and weight of 2 mg AS RDSTs was shown to affect influx of atropine sulfate.

The herein described atropine sulfate (AS) rapidly-disintegrating sublingual tablets (RDSTs) provide a non-invasive sublingual drug delivery of atropine sulfate for the treatment of acute organophosphate (OP) toxicity as a potential alternative (to the conventional parenteral delivery), patient-friendly, convenient, and cost-effective dosage form.

The following items represent further embodiments of the description:

Item 1. A pharmaceutical composition comprising atropine sulfate and at least one pharmaceutically-acceptable excipient, the composition formulated for rapid disintegration in buccal or sublingual administration.

Item 2. The pharmaceutical composition in accordance with Item 1, wherein the at least one pharmaceutically-acceptable excipient is selected from the group consisting of a filler, a superdisintegrant, and a lubricant.

Item 3. The pharmaceutical composition in accordance with Item 2, comprising a filler, a superdisintegrant, and a

lubricant, wherein the filler is microcrystalline cellulose, the superdisintegrant is hydroxypropyl cellulose, and the lubricant is magnesium stearate.

Item 4. The pharmaceutical composition in accordance with Item 3, wherein the hydroxypropyl cellulose is low substituted.

Item 5. The pharmaceutical composition in accordance with Item 3, wherein the composition comprises from about 2 to about 20 % of atropine sulfate, from about 20 to about 95% of microcrystalline cellulose, from about 1 to about 15 % of hydroxypropyl cellulose, and from about 0.5 to about 3 % of magnesium stearate.

Item 6. The pharmaceutical composition in accordance with Item 3, wherein the composition comprises from about 1 to about 20 wt% of atropine sulfate, from about 20 to about 95 wt% of microcrystalline cellulose, from about 1 to about 15 wt% hydroxypropyl cellulose, and from about 0.5 to about 3 wt% magnesium stearate.

Item 7. A method for manufacturing atropine sulfate tablets formulated for rapid disintegration in buccal or sublingual administration, the method comprising:

weighing atropine sulfate and pharmaceutically-acceptable excipients;
sieving the atropine sulfate and pharmaceutically-acceptable excipients;
combining and mixing the atropine sulfate and pharmaceutically-acceptable excipients to form a directly compressible formulation; and
directly compressing the formulation to form the atropine sulfate tablets.

Item 8. The method in accordance with Item 7, wherein the formulation is directly compressed using concave punches and dies.

Item 9. The method in accordance with Item 7, wherein the pharmaceutically-acceptable excipients include a filler, a superdisintegrant, and a lubricant.

Item 10. The method in accordance with Item 9, wherein the filler is microcrystalline cellulose, the superdisintegrant is hydroxypropyl cellulose, and the lubricant is magnesium stearate.

Item 11. The method in accordance with Item 10, wherein the hydroxypropyl cellulose is low substituted.

Item 12. The method in accordance with Item 10, wherein the combining and mixing comprises the steps of:

a) combining the atropine sulfate with the microcrystalline cellulose and mixing to form a mixture;
b) adding two thirds of the hydroxypropyl cellulose to the mixture formed in step a) and mixing for about four minutes to form a mixture;
c) mixing the magnesium stearate and the remaining one third of the hydroxypropyl cellulose to form a second mixture; and
d) adding the second mixture to the mixture formed in step b) and mixing for about thirty seconds.

Item 13. The method in accordance with Item 12, wherein the formulation is directly compressed using concave punches and dies.

Item 14. Atropine sulfate tablets formulated for rapid disintegration in buccal or sublingual administration produced in accordance with the method of Item 13. Item 15.
A method for treating symptoms of exposure to organophosphates (OPs) in a subject in need thereof, comprising:

providing a composition including atropine sulfate and at least one pharmaceutically-acceptable excipient, the composition formulated for rapid disintegration in buccal or sublingual administration; and
administering the composition to the subject.

Item 16. The method in accordance with Item 15, wherein a source of the exposure is pesticides or nerve agents.

Item 17. The method in accordance with Item 15, wherein the at least one pharmaceutically-acceptable excipient is selected from the group consisting of a filler, a superdisintegrant, and a lubricant.

Item 18. The method in accordance with Item 17, comprising a filler, a superdisintegrant, and a lubricant, wherein the filler is microcrystalline cellulose, the superdisintegrant is hydroxypropyl cellulose, and the lubricant is magnesium stearate.

Item 19. The pharmaceutical composition in accordance with Item 18, wherein the hydroxypropyl cellulose is low substituted.

Item 20. The method in accordance with Item 15, further comprising administering a cholinesterase re-activator concomitantly with the composition.

Item 21. The method in accordance with Item 20, wherein the cholinesterase re-activator is pralidoxime chloride.

Item 22. A composition comprising atropine sulfate (AS) and at least one pharmaceutically-acceptable excipient for use in the manufacture of a composition for treating symptoms of exposure to organophosphates (OPs).

Item 23. Use in accordance with Item 22, wherein a source of the exposure is pesticides or nerve agents.

Item 24. Use in accordance with Item 22, wherein the at least one pharmaceutically-acceptable excipient is selected from the group consisting of a filler, a superdisintegrant, and a lubricant.

Item 25. A composition comprising atropine sulfate (AS), a filler, a superdisintegrant, and a lubricant for use in the manufacture of a composition for treating symptoms of exposure to organophosphates (OPs).

Item 26. Use in accordance with Item 25, wherein the filler is microcrystalline cellulose, the superdisintegrant is hydroxypropyl cellulose, and the lubricant is magnesium stearate.

Item 27. Use in accordance with Item 26, wherein the hydroxypropyl cellulose is low substituted.

Item 28. A composition comprising atropine sulfate (AS), at least one pharmaceutically-acceptable excipient, and a cholinesterase re-activator for use in the manufacture of a composition for treating symptoms of exposure to organophosphates (OPs).

Item 29. Use in accordance with Item 28, wherein the cholinesterase re-activator is pralidoxime chloride.

## Claims

1. A rapidly-disintegrating sublingual tablet (RDST) comprising atropine sulfate, a filler, a superdisintegrant and a lubricant.

2. The rapidly-disintegrating sublingual tablet (RDST) according to Claim 1, wherein the filler is microcrystalline cellulose, the superdisintegrant is hydroxypropyl cellulose, and the lubricant is magnesium stearate.

3. The rapidly-disintegrating sublingual tablet (RDST) according to Claim 2, wherein the hydroxypropyl cellulose is low substituted.

4. The rapidly-disintegrating sublingual tablet (RDST) according to Claim 2, wherein the composition comprises from 2 to 20% of atropine sulfate, from 20 to 95% of microcrystalline cellulose, from 1 to 15% of hydroxypropyl cellulose, and from 0.5 to 3% of magnesium stearate.

5. The rapidly-disintegrating sublingual tablet (RDST) according to Claim 2, wherein the composition comprises from 1 to 20 wt% of atropine sulfate, from 20 to 95 wt% of microcrystalline cellulose, from 1 to 15 wt% of hydroxypropyl cellulose, and from 0.5 to 3wt% of magnesium stearate.

6. A rapidly-disintegrating sublingual tablet (RDST) according to any one of the preceding claims, said tablet is for use for sublingual administration in the treatment of symptoms of exposure to organophosphates.

7. The rapidly-disintegrating sublingual tablet (RDST) according to Claim 6, said tablet is for use for sublingual administration in the treatment of symptoms of exposure to pesticides or nerve agents.

## Patentansprüche

1. Schnell zerfallende sublinguale Tablette (RDST) mit Atropinsulfat, einem Füllstoff, einem Supersprengmittel und einem Gleitmittel.

2. Schnell zerfallende sublinguale Tablette (RDST) nach Anspruch 1, wobei der Füllstoff mikrokristalline Cellulose ist, das Supersprengmittel Hydroxypropylcellulose ist und das Gleitmittel Magnesiumstearat ist.

3. Schnell zerfallende sublinguale Tablette (RDST) nach Anspruch 2, wobei die Hydroxypropylcellulose niedrig substituiert ist.

4. Schnell zerfallende sublinguale Tablette (RDST) nach Anspruch 2, wobei die Zusammensetzung 2 bis 20 % Atropinsulfat, 20 bis 95 % mikrokristalline Cellulose, 1 bis 15 % Hydroxypropylcellulose und 0,5 bis 3 % Magnesiumstearat umfasst.

5. Schnell zerfallende sublinguale Tablette (RDST) nach Anspruch 2, wobei die Zusammensetzung 1 bis 20 Gew.-% Atropinsulfat, 20 bis 95 Gew.-% mikrokristalline Cellulose, 1 bis 15 Gew.-% Hydroxypropylcellulose und 0,5 bis 3 Gew.-% Magnesiumstearat umfasst.

6. Schnell zerfallende sublinguale Tablette (RDST) nach einem der vorangehenden Ansprüche, wobei die Tablette zur Verwendung für die sublinguale Verabreichung bei der Behandlung von Symptomen der Einwirkung von Organophosphaten dient.

7. Schnell zerfallende sublinguale Tablette (RDST) nach Anspruch 6, wobei die Tablette zur Verwendung für die sublinguale Verabreichung bei der Behandlung von Symptomen der Einwirkung von Pestiziden oder Nervengasen dient.

## Revendications

1. Comprimé sublingual à désintégration rapide (RDST) comprenant du sulfate d'atropine, une charge, un super-désintégrant et un lubrifiant.

2. Comprimé sublingual à désintégration rapide (RDST) selon la revendication 1, dans lequel la charge est une cellulose microcristalline, le super-désintégrant est une hydroxypropyl cellulose, et le lubrifiant est le stéarate de magnésium.

3. Comprimé sublingual à désintégration rapide (RDST) selon la revendication 2, dans lequel l'hydroxypropyl cellulose est faiblement substituée.

4. Comprimé sublingual à désintégration rapide (RDST) selon la revendication 2, dans lequel la composition comprend de 2 à 20 % de sulfate d'atropine, de 20 à 95 % de cellulose microcristalline, de 1 à 15 % d'hydroxypropyl cellulose, et de 0,5 à 3 % de stéarate de magnésium.

5. Comprimé sublingual à désintégration rapide (RDST) selon la revendication 2, dans lequel la composition comprend de 1 à 20 % en poids de sulfate d'atropine, de 20 à 95 % en poids de cellulose microcristalline, de 1 à 15 % en poids d'hydroxypropyl cellulose, et de 0,5 à 3 % en poids de stéarate de magnésium.

6. Comprimé sublingual à désintégration rapide (RDST) selon l'une quelconque des revendications précédentes, ledit comprimé est pour une utilisation pour une administration sublinguale dans le traitement des symptômes d'une exposition à des organophosphates.

7. Comprimé sublingual à désintégration rapide (RDST) selon la revendication 6, ledit comprimé est pour une utilisation pour une administration sublinguale dans le traitement des symptômes d'une exposition à des pesticides ou des agents neurotoxiques.

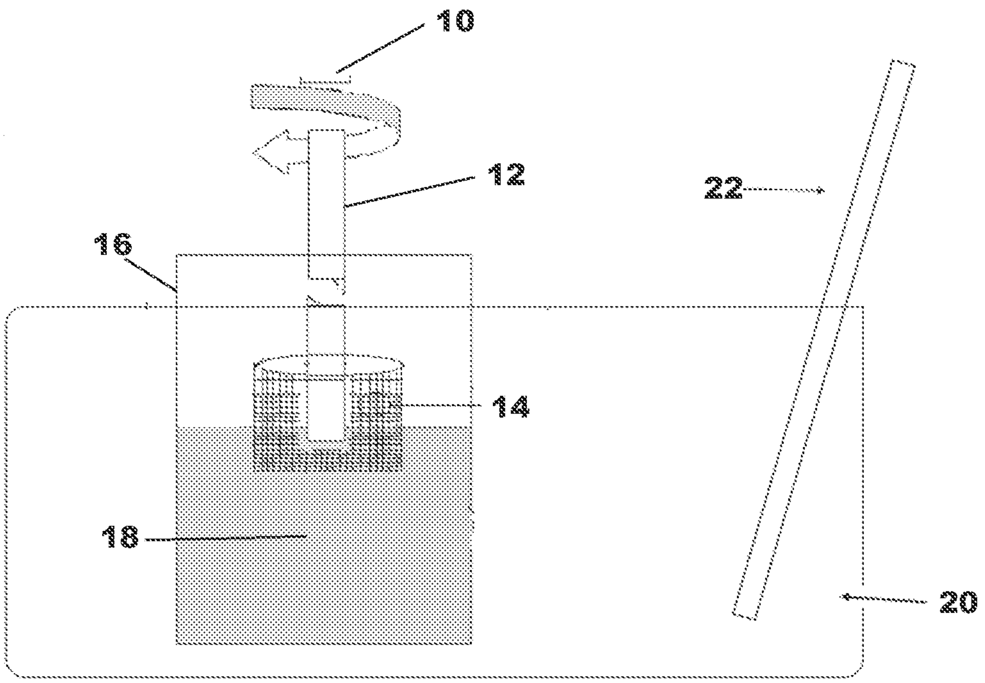


FIG. 1

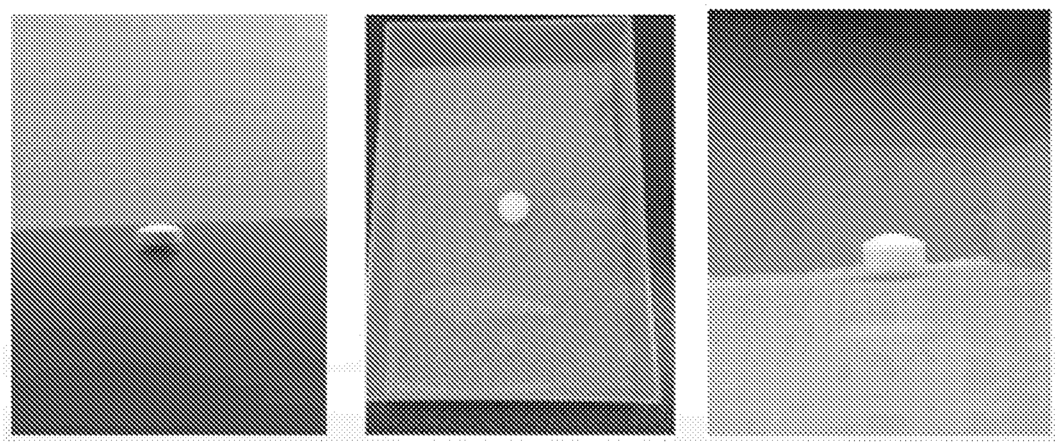

FIG. 2

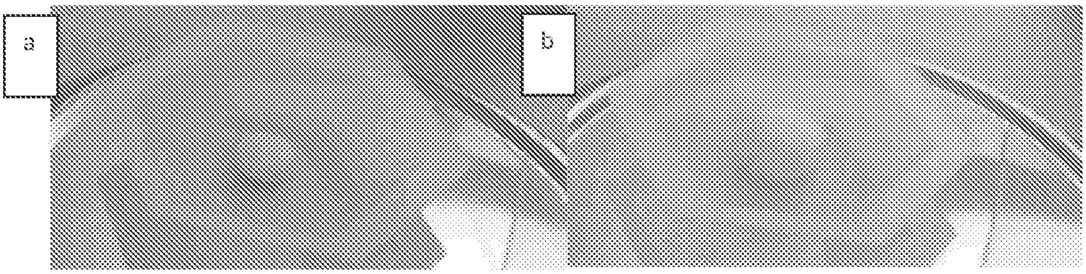
a
b

FIG. 3

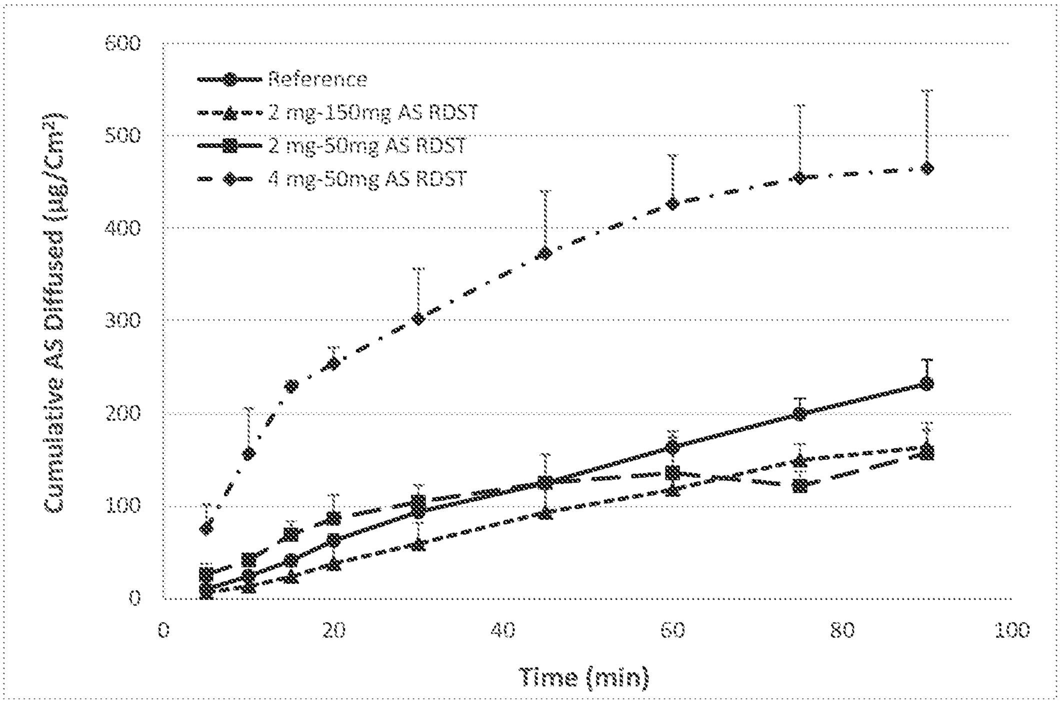
Reference
2 mg-150mg AS RDST
2 mg-50mg AS RDST
4 mg-50mg AS RDST
Cumulative AS Diffused (μg/Cm²)
Time (min)
0
100
200
300
400
500
600
0
20
40
60
80
100

FIG. 4

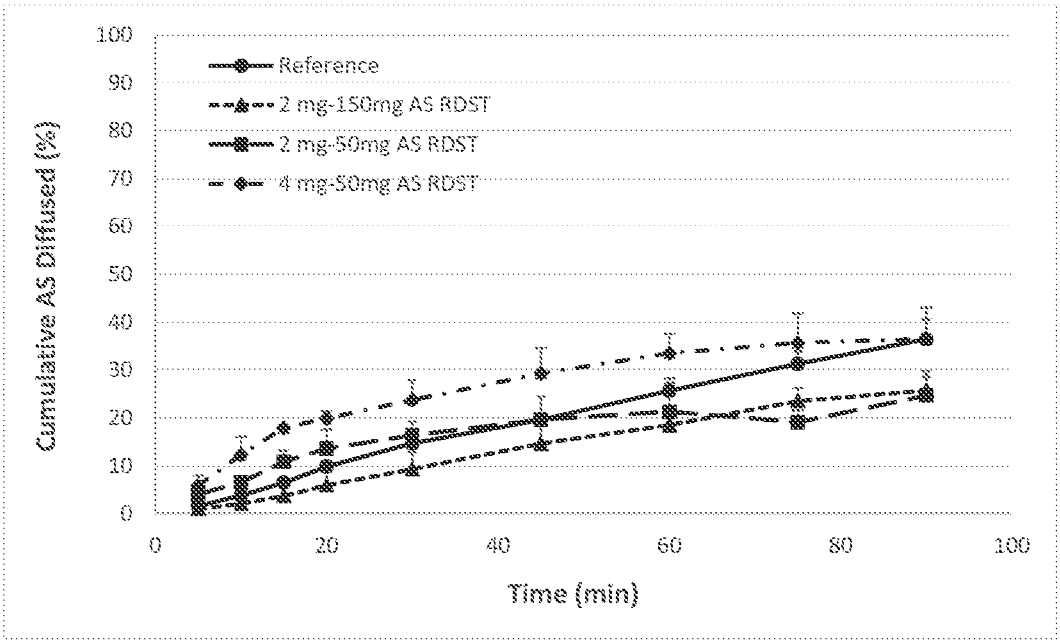
Cumulative AS Diffused (%)
100
90
80
70
60
50
40
30
20
10
0
0
20
40
60
80
100
Time (min)
Reference
2 mg-150mg AS RDST
2 mg-50mg AS RDST
4 mg-50mg AS RDST

FIG. 5

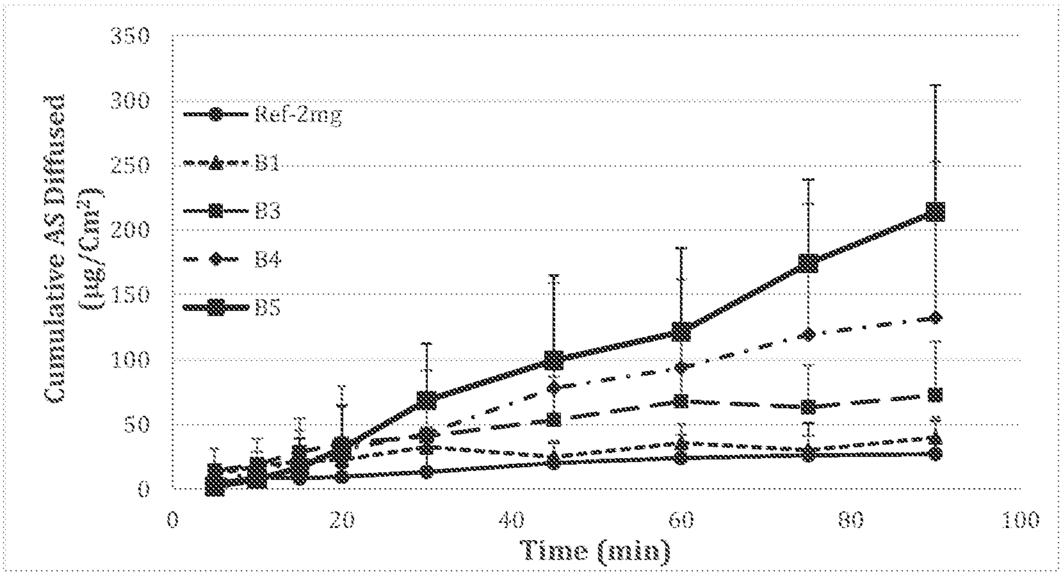

Cumulative AS Diffused ($\mu g/Cm^2$)
Time (min)
Ref-2mg
B1
B3
B4
B5
0
50
100
150
200
250
300
350
0
20
40
60
80
100


FIG. 6

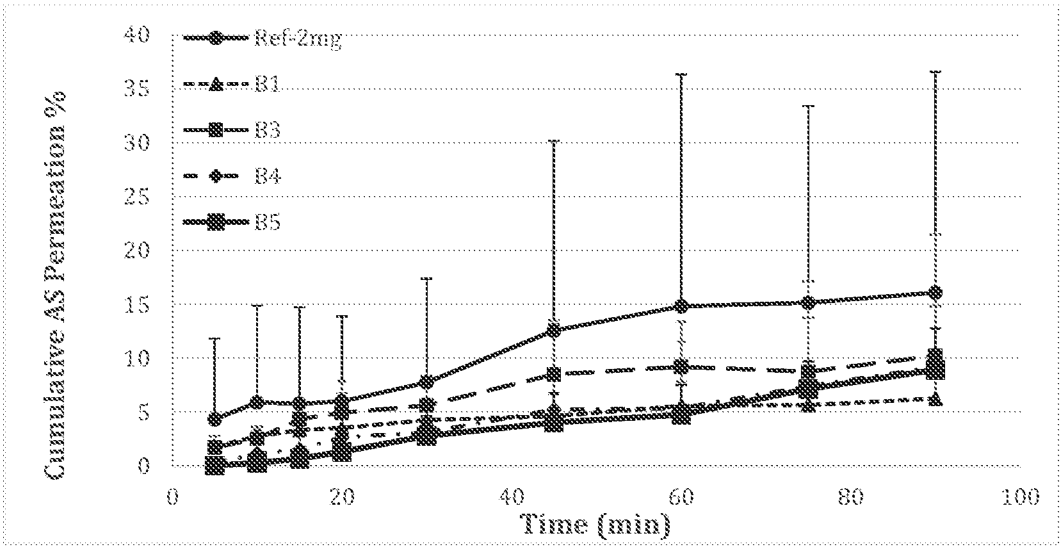

Cumulative AS Permeation %
Time (min)
Ref-2mg
B1
B3
B4
B5
0
5
10
15
20
25
30
35
40
0
20
40
60
80
100


FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **NEWMARK.** *J. Neurology,* 2004, vol. 62, 1590-1596
- **EDDLESTON M. et al.** *Crit Care,* 2004, vol. 8, R391-R7
- **BUCKLEY N. et al.** *J Toxicol Clin Toxicol,* 1994, vol. 32, 61-68
- **EDDLESTON M. et al.** *Lancet,* 2008, vol. 371, 597-607
- The Impact of Pesticides on Health: Preventing Intentional and Unintentional Deaths from Pesticide Poisoning. **BERTOLOTE J. et al.** Prevention, Pesticides and Health. World Health Organization (WHO), 2004
- Pesticide Usage in the United States: History, Benefits, Risks, and Trends. NC Cooperative Extension. North Carolina State University website, 1996
- **BRONSTEIN A. et al.** *Clin Toxicol,* 2012, vol. 50, 911-1164
- Weapons of Mass Destruction: Chemical Weapons. United Nations Office for Disarmament Affairs website
- **CRODDY E. et al.** Weapons of Mass Destruction: An Encyclopedia of Worldwide Policy, Technology, and History. ABC-CLIO, 2005
- **NEWMARK J.** *Neurology,* 2004, vol. 62, 1590-1596
- **BENTUR Y. et al.** *Clin Toxicol,* 2006, vol. 44, 301-306
- United Nations Mission to Investigate Allegations of the Use of Chemical Weapons in the Syrian Arab Republic. Report on the Alleged Use of Chemical Weapons in the Ghouta Area of Damascus. United Nations website, 21 August 2013
- **RAWAS-QALAJI M. et al.** *J Allergy Clin Immun,* 2006, vol. 117, 398-403
- **CORCORAN T. et al.** *J Aerosol Med Pulm D,* 2013, vol. 26, 46-55
- **HAGUE J. et al.** *Mil Med,* 2004, vol. 169, 389-391
- **BIRUDARAJ R. et al.** *J Pharm Sci,* 2005, vol. 94, 70-78
- **MOTWANI J. et al.** *Clin Pharmacokinet,* 1991, vol. 21 (2), 83-94
- **ISHIKAWA T.** *Chem Pharm Bull,* 2001, vol. 49, 230-232
- **PRICE T. et al.** *Obstet Gynecol,* 1997, vol. 89, 340-345
- **KROBOTH P. et al.** *J Clin Psychopharmacol,* 1995, vol. 15 (4), 259-262
- **CUNNINGHAM F. et al.** *J Clin Anesth,* 1994, vol. 6, 430-433
- **SCAVONE J. et al.** *Eur J Clin Pharmacol,* 1992, vol. 42, 439-443
- **SPENARD J. et al.** *Biopharm Drug Dispos,* 1988, vol. 9, 457-464
- **MITRA A. et al.** *Encyclopedia of Pharm Tech,* 2002, 2081-2095
- **DE VARIES et al.** *Crit Rev Ther Drug Carr Syst.,* 1991, vol. 8, 271-303
- **DOLGIN E.** *Nat Med,* 2013, vol. 19, 1194-1195
- **RAMARAO G. et al.** *BMC Neuroscience,* 2014, vol. 15, 47
- **RAIPAL et al.** *American Journal of Emergency Medicine,* 2010, vol. 28, 143-150
- **BREDFORD et al.** *Academic Emergency Medicine,* 2003, vol. 10 (3), 286-288
- **MANGAL, M. et al.** *International Journal of Pharmacy and Pharmaceutical Science Research,* 2012, vol. 2 (2), 26-35
- **KUMAR G. et al.** *Journal of Global Pharma Technology,* 2012, vol. 4 (02), 1-12
- United States Pharmacopeia. United States Pharmacopeia Convention, Inc, 2014, 1878-1879
- United States Pharmacopeia. United States Pharmacopeia Convention, Inc, 2014, 491-494
- United States Pharmacopeia. United States Pharmacopeia Convention, Inc, 2014, 1146-1148
- United States Pharmacopeia. United States Pharmacopeia Convention, Inc, 2014, 1145-1146
- Disintegration. U.S. Pharmacopeia. 17 September 2014
- **RAWAS-QALAJI M. et al.** *Drug Dev Ind Pharm,* 2007, vol. 33, 523-530
- **RAWAS-QALAJI M. et al.** *AAPS PharmSciTech,* 2006, vol. 7, E41
- **SHUKLA, D. et al.** *Sci Pharm,* 2009, vol. 77, 327-341
- **RACHID O. et al.** *AAPS PharmSciTech,* 2011, vol. 12, 544-552
- United States Pharmacopeia. United States Phamacopeial Convention, Inc, 2014, 1875-1876
- *AAPS PharmSciTech.,* 2006, vol. 7 (2), E72-E8
- Comparative structure of porcine oral epithelium. Journal of Dental Research. 1981

- **BIRUDARAJ R ; BERNER B ; SHEN S ; LI X.** Buccal permeation of buspirone: mechanistic studies on transport pathways. *Journal Of Pharmaceutical Sciences,* 2005, vol. 94 (1), 70-8
- *International Journal of Pharmaceutics,* 2009, vol. 366 (1-2), 58-64